# EUROPEAN PATENT APPLICATION

(11) **EP 4 667 579 A1**
(43) Date of publication of application: **24.12.2025**
(21) Application number: 24771185.6
(22) Date of filing: 13.03.2024
(51) Int. Cl.: C12P 7/62, C12N 15/74, C12N 1/20, C12N 9/00

(54) **METHANOTROPH FOR PRODUCING PHB, AND PHB PRODUCTION METHOD USING SAME**

(30) Priority: 15.03.2023 KR 20230034023
(71) Applicant: CJ CheilJedang Corporation, Seoul 04560 (KR)
(72) Inventor: LEE, Kyung-Chang, Seoul 04560 (KR); NAM, Ji Won, Seoul 04560 (KR); KIM, Sujin, Seoul 04560 (KR)
(74) Representative: Meissner Bolte Partnerschaft mbB
(86) International application number: PCT/KR2024/003218
(87) International publication number: WO 2024/191186

(57) **Abstract**

The present application relates to: a methanotroph having enhanced PHB productivity through a malate thiokinase activity that is greater than the intrinsic activity thereof, a PHB production method comprising a step of culturing the methanotroph in a medium; a PHB production composition comprising the methanotroph, a culture of the methanotroph, a fermentation product of the methanotroph or a combination of at least two thereof; and a PHB production use of the methanotroph.

## Description

### [Technical Field]

The present disclosure relates to a methanotrophic bacterium having a PHB-producing ability, in which the activity of malate thiokinase is enhanced, as compared to its endogenous activity; a method of producing PHB, the method comprising the step of culturing the methanotrophic bacterium in a medium; a composition for producing PHB, the composition comprising the methanotrophic bacterium, a culture of the methanotrophic bacterium, a fermentation product of the methanotrophic bacterium, or a combination of two or more thereof; and use of the methanotrophic bacterium in producing PHB.

### [Background Art]

Methane is a non-carbon dioxide greenhouse gas, and although its atmospheric concentration is low, its global warming potential is about 84 times higher than that of carbon dioxide (CO₂), accounting for about 15% to about 20% of the causes of global warming. Methanotrophic bacteria are methanotrophic bacteria that utilize methane as a carbon source and energy source, and their usefulness is emerging due to their environmental and economic advantages. However, due to the lack of understanding of their physiological characteristics and metabolic regulation, the technical difficulty of research and industrialization using the same is high.

Polyhydroxybutyrate (PHB), a type of biodegradable plastic, is a polymeric material that is accumulated by methanotrophic bacteria as a carbon source and energy storage material, when nutrients such as nitrogen or phosphorus, etc. are limited in the presence of a large amount of carbon source. Polyhydroxybutyrate has mechanical properties similar to those of several currently, commercially available petroleum-based synthetic plastics, and is receiving attention as an alternative to petroleum-based synthetic polymers due to its property of being completely degraded by methanotrophic bacteria in the natural environment.

In this regard, Korean Patent No. 10-2202694 discloses a methanol-assimilating bacterium (or methylotrophic bacterium) mutant strain overexpressing the formate-tetrahydrofolate ligase (ftfL) gene and a method of producing PHB using the same. However, there is still a need for research on a method of efficiently producing PHB using methanotrophic bacteria.

Accordingly, there is still a need for research to effectively increase PHB-producing ability.

### [Disclosure]

### [Technical Problem]

The present inventors found that a methanotrophic bacterium, in which the activity of malate thiokinase is enhanced, as compared to its endogenous activity, is able to produce PHB, thereby completing the present disclosure.

### [Technical Solution]

An aspect of the present disclosure provides a methanotrophic bacterium having a PHB-producing ability, in which the activity of malate thiokinase is enhanced, as compared to its endogenous activity.

In one specific embodiment, the enhancement may be enhancement of the intracellular copy number; modification of a gene expression regulatory region; modification of a nucleotide sequence encoding a start codon or 5'-UTR region; modification of an amino acid sequence of a polypeptide; modification of a polynucleotide sequence; introduction of a foreign polypeptide or a foreign polynucleotide encoding the same; codon optimization of a polynucleotide; structural modification of a polypeptide; or a combination thereof.

In one specific embodiment, the malate thiokinase may be any one or more proteins selected from the group consisting of MtkA and MtkB.

With regard to the methanotrophic bacterium according to any one of the aforementioned specific embodiments, the MtkA protein may consist of an amino acid sequence of SEQ ID NO: 1, and the MtkB protein may consist of an amino acid sequence of SEQ ID NO: 2.

With regard to the methanotrophic bacterium according to any one of the aforementioned specific embodiments, the methanotrophic bacterium may be a methanotrophic bacterium of the genus *Methylocystis* (*Methylocystis* sp.).

With regard to the methanotrophic bacterium according to any one of the aforementioned specific embodiments, the methanotrophic bacterium has an increased PHB-producing ability, as compared to an unmodified methanotrophic bacterium.

Another aspect of the present disclosure provides a method of producing PHB, the method comprising the step of culturing, in a medium, a methanotrophic bacterium having a PHB-producing ability, in which the activity of malate thiokinase is enhanced, as compared to its endogenous activity.

In one specific embodiment, the step of recovering PHB from the cultured methanotrophic bacterium, a culture of the methanotrophic bacterium, a fermentation product of the methanotrophic bacterium, or the culture medium may be further comprised.

Still another aspect of the present disclosure provides a composition for producing PHB, the composition comprising a methanotrophic bacterium having a PHB-producing ability, in which the activity of malate thiokinase is enhanced, as compared to its endogenous activity, a culture of the methanotrophic bacterium, a fermentation product of the methanotrophic bacterium, or a combination of two or more thereof.

Still another aspect of the present disclosure provides use of a methanotrophic bacterium, in which the activity of malate thiokinase is enhanced, as compared to its endogenous activity, in producing PHB.

### [Advantageous Effects]

A methanotrophic bacterium of the present disclosure, in which the activity of malate thiokinase is enhanced, as compared to its endogenous activity, is able to produce PHB in a high yield, thereby being usefully applied to the industrial production of PHB.

### [Brief Description of the Drawing]

FIG. 1 is a diagram showing the cell growth of an MtkAB-enhanced strain of the present disclosure; and
FIG. 2 is a diagram showing the PHB-producing ability of the MtkAB-enhanced strain of the present disclosure.

### [Detailed Description of Preferred Embodiments]

The present disclosure will be described in detail as follows. Meanwhile, each description and embodiment disclosed in this disclosure may also be applied to other descriptions and embodiments. That is, all combinations of various elements disclosed in this disclosure fall within the scope of the present disclosure. Further, the scope of the present disclosure is not limited by the specific description described below.

Further, those skilled in the art will recognize, or be able to ascertain using no more than routine experimentation, many equivalents to the specific embodiments of the disclosure described herein. Further, these equivalents should be interpreted to fall within the present disclosure.

As used in the specification and appended claims of the present disclosure, the singular forms ("a", "an", and "the") comprise plural referents unless the context clearly states otherwise. Unless the context indicates otherwise, singular terms shall comprise the plural, and plural terms shall comprise the singular. As used in the specification and appended claims of the present disclosure, unless stated otherwise, the use of "or" may be used to comprise "and/or".

As used herein, the term "about" may be presented before a particular numerical value. The term "about" used herein comprises not only the exact number recited after the term, but also a range that is near or close to that number. Considering the context in which the number is presented, it may be determined whether any number is close to or near the particular number presented. In one example, the term "about" may refer to a range from -10% to +10% of a numerical value. In another example, the term "about" may refer to a range from -5% to +5% of a given numerical value, but is not limited thereto.

As used herein, the descriptions such as the terms "first, second, third,," "i), ii), iii),," or "(a), (b), (c), (d),," are used to distinguish similar constitutions, and these terms do not mean that the constitutions are performed continually or sequentially. For example, when the terms are used in reference to steps of a method, use, or assay, there may be no time interval between these steps, or they may be performed concurrently, or may be performed several seconds, several minutes, several hours, several days, or several months apart.

As used herein, the term "consisting essentially of" may mean that, in the case where the features of the object claimed herein are not substantially affected by the presence of an unspecified component, the unspecified component may be present.

As used herein, the term "consisting of" means that the total ratio of specific component(s) is 100%. The components or features that are recited after the term "consisting of" may be essential or mandatory. In some embodiments, in addition to the components or features recited after the term "consisting of", any other components or non-essential components may be excluded.

As used herein, the term "comprising" means the presence of features, steps or components recited after the term, and does not exclude the presence or addition of one or more features, steps or components. The components or features recited after the term "comprising" herein may be essential or mandatory. However, in some embodiments, the term may further comprise any other or non-essential components or features.

An aspect of the present disclosure provides a methanotrophic bacterium having a PHB-producing ability, in which the activity of malate thiokinase is enhanced, as compared to its endogenous activity.

The enhanced activity of malate thiokinase may be defined as, but is not limited to, increased PHB-producing ability of the methanotrophic bacterium of the present disclosure, as compared to the PHB-producing ability of a native wild-type methanotrophic bacterium or an unmodified methanotrophic bacterium (e.g., a methanotrophic bacterium expressing the polypeptide having the wild-type malate thiokinase activity (e.g., the polypeptide of SEQ ID NO: 1 and/or SEQ ID NO: 2) or a strain in which the activity of malate thiokinase of the present disclosure is not enhanced, or has not been enhanced, as compared to the endogenous activity).

For example, the activity of malate thiokinase may be determined by measuring the PHB-producing ability or yield, but is not limited thereto.

As used herein, the term "malate thiokinase" is a bacterial enzyme that forms malyl-CoA from malate and CoA, consisting of two different subunits. The malate thiokinase of the present disclosure may be any one or more proteins selected from the group consisting of MtkA and MtkB, but is not limited thereto. Specifically, the malate thiokinase of the present disclosure may be used to mean a complex of MtkA and MtkB, and may be used interchangeably with MtkAB, malate-CoA ligase, or succinate-CoA ligase.

Specifically, the malate thiokinase of the present disclosure may be a protein having activities of MtkA and MtkB proteins, each encoded by mtkA and mtkB genes, but is not particularly limited to its type as long as it has the activity corresponding to malate thiokinase. The MtkA and MtkB proteins, each encoded by mtkA and mtkB genes, are known in the art, and amino acid and polynucleotide sequences of the MtkA and MtkB proteins may be obtained from known databases, for example, NCBI GenBank, etc., but are not limited thereto.

For example, the MtkA and MtkB proteins of the present disclosure may comprise the amino acid sequences of SEQ ID NO: 1 and SEQ ID NO: 2, respectively, or amino acid sequences having at least 60% homology or identity thereto, but are not limited thereto, as long as they have the activity of malate thiokinase. Specifically, the polypeptide having the MtkA and MtkB protein activities may have or comprise SEQ ID NO: 1 and SEQ ID NO: 2 or amino acid sequences having at least 60%, 70%, 80%, 85%, 90%, 95%, 96%, 97%, 98%, or 99% or more homology or identity to SEQ ID NO: 1 and SEQ ID NO: 2, or may consist of the amino acid sequences, or may essentially consist of the amino acid sequences. For example, the MtkA and MtkB proteins may refer to proteins that endogenously exist in methanotrophic bacteria, but are not limited thereto. Specifically, the MtkA and MtkB proteins may be MtkA protein consisting of the amino acid sequence of SEQ ID NO: 1 and MtkB protein consisting of the amino acid sequence of SEQ ID NO: 2, which endogenously exist in methanotrophic bacteria, but are not limited thereto.

With respect to amino acid sequences in the present disclosure, although it is described as a polypeptide "comprising" an amino acid sequence described by a specific sequence number, a polypeptide "consisting of" an amino acid sequence described by a specific sequence number, or a polypeptide or protein "having" an amino acid sequence described by a specific sequence number, it is apparent that any protein having an amino acid sequence in which part of the sequence is deleted, modified, substituted, conservatively substituted, or added may fall within the scope of the present disclosure as long as it has the identical or corresponding activity to that of the protein consisting of the amino acid sequence of the corresponding sequence number. For example, as long as a protein has the identical or corresponding activity to that of the modified protein, it does not exclude the addition of a sequence that does not change the function of the protein before or after the amino acid sequence, a naturally occurring mutation, a silent mutation thereof, or conservative substitution, and it is apparent that even though the protein has such sequence additions or mutations, it falls within the scope of the present disclosure.

For example, it may be a case of having the addition of a sequence that does not change the function of the variant polypeptide of the present disclosure at the N-terminus, C-terminus, and/or inside of the amino acid sequence, a naturally occurring mutation, a silent mutation, or a conservative substitution. For example, the polypeptide may be conjugated to an N-terminal signal (or leader) sequence of a protein that is co-translationally or post-translationally involved in protein transfer. Further, the polypeptide may be conjugated to another sequence or linker so as to enable identification, purification, or synthesis of the polypeptide.

As used herein, the term "conservative substitution" refers to substitution of an amino acid with another amino acid having similar structural and/or chemical properties. Such amino acid substitution may generally occur based on similarity of polarity, charge, solubility, hydrophobicity, hydrophilicity, and/or amphipathic nature of a residue. For example, positively charged (basic) amino acids comprise arginine, lysine, and histidine; negatively charged (acidic) amino acids comprise glutamic acid and aspartic acid; amino acids having a nonpolar side chain (nonpolar amino acids) comprise glycine, alanine, valine, leucine, isoleucine, methionine, phenylalanine, tryptophan, and proline; amino acids having a polar or hydrophilic side chain (polar amino acids) comprise serine, threonine, cysteine, tyrosine, asparagine, and glutamine. For another example, amino acids may be classified into amino acids with electrically charged side chains (electrically charged amino acids) such as arginine, lysine, histidine, glutamic acid, and aspartic acid, and amino acids with uncharged side chains (uncharged amino acids; also called neutral amino acids) such as glycine, alanine, valine, leucine, isoleucine, methionine, phenylalanine, tryptophan, proline, serine, threonine, cysteine, tyrosine, asparagine, and glutamine. For still another example, phenylalanine, tryptophan, and tyrosine may be classified as aromatic amino acids. For still another example, valine, leucine, and isoleucine may be classified as branched amino acids. For still another example, the 20 types of amino acids are classified according to size, starting from the amino acid group with relatively small volume, the amino acids may be classified into five groups; glycine, alanine, serine; cysteine, proline, threonine, aspartic acid, asparagine; valine, histidine, glutamic acid, glutamine; isoleucine, leucine, methionine, lysine, arginine; and phenylalanine, tryptophan, tyrosine. However, they are not necessarily limited thereto. Usually, conservative substitution may hardly affect or not affect the activity of polypeptides.

Further, a nucleotide sequence encoding the malate thiokinase may be a nucleotide sequence encoding a protein that exhibits the activity of malate thiokinase.

For example, the nucleotide sequences encoding the MtkA and MtkB proteins may be a nucleotide sequence encoding the protein exhibiting the activity of malate thiokinase.

For example, the MtkA and MtkB proteins, each having the amino acid sequences of SEQ ID NO: 1 and SEQ ID NO: 2, may be encoded by polynucleotides each having or comprising the nucleotide sequences of SEQ ID NO: 3 and SEQ ID NO: 4 or nucleotide sequences having 60% or more, 70% or more, 75% or more, 80% or more, 85% or more, 90% or more, 95% or more, 96% or more, 97% or more, 98% or more, and less than 100% homology or identity to the nucleotide sequences of SEQ ID NO: 3 and SEQ ID NO: 4, or consisting of or essentially consisting of the nucleotide sequences, but are not limited thereto. Further, the nucleotide sequences of SEQ ID NO: 3 and SEQ ID NO: 4 may be obtained from known databases, for example, NCBI GenBank, etc., but are not limited thereto.

In the present disclosure, for example, the gene comprising the nucleotide sequence of SEQ ID NO: 3 may be used interchangeably with a polynucleotide comprising the nucleotide sequence of SEQ ID NO: 3, a gene or polynucleotide having the nucleotide sequence of SEQ ID NO: 3, and a gene or polynucleotide consisting of the nucleotide sequence of SEQ ID NO: 3.

The polynucleotide of the present disclosure may undergo various modifications in the coding region without changing the amino acid sequence of the malate thiokinase of the present disclosure due to codon degeneracy or in consideration of the codons preferred in an organism that is intended to express the malate thiokinase of the present disclosure. Therefore, it is apparent that a polynucleotide which may be translated to the polypeptide consisting of the amino acid sequence of the malate thiokinase of the present disclosure or a polypeptide having homology or identity thereto due to codon degeneracy may also be comprised. For example, the polynucleotide of the present disclosure may be SEQ ID NO: 3 and/or SEQ ID NO: 4 or a degenerated sequence thereof.

For another example, the polynucleotide of the present disclosure may have or comprise a nucleotide sequence having 60% or more, 70% or more, 75% or more, 80% or more, 85% or more, 90% or more, 95% or more, 96% or more, 97% or more, 98% or more, and less than 100% homology or identity to SEQ ID NO: 3 and/or SEQ ID NO: 4, or may consist of or essentially consist of a nucleotide sequence having 60% or more, 70% or more, 75% or more, 80% or more, 85% or more, 90% or more, 95% or more, 96% or more, 97% or more, 98% or more, and less than 100% homology or identity to SEQ ID NO: 3 and/or SEQ ID NO: 4, but is not limited thereto.

Further, the polynucleotide of the present disclosure may comprise a probe which may be prepared from a known gene sequence, for example, any sequence without limitation as long as it hybridizes with a sequence complementary to the entirety or a part of the polynucleotide sequence of the present disclosure under stringent conditions to encode the malate thiokinase of the present disclosure.

As used herein, the term "homology" or "identity" refers to a degree of relevance between two given amino acid sequences or nucleotide sequences, and may be expressed as a percentage. The terms homology and identity may often be used interchangeably with each other.

The sequence homology or identity of a conserved polynucleotide or polypeptide is determined by standard alignment algorithms, and the default gap penalty established by a program to be used may be used together. Substantially, homologous or identical sequences may generally hybridize under moderately or highly stringent conditions to the entirety of the sequence or at least about 50%, 60%, 70%, 80%, or 90% of the full-length. It is apparent that hybridization also comprises hybridization of a polynucleotide with a polynucleotide comprising a general codon or a codon in consideration of codon degeneracy.

Whether any two polynucleotide or polypeptide sequences have homology, similarity, or identity may be determined using known computer algorithms such as the "FASTA" program, for example, using default parameters as in Pearson et al (1988) [Proc. Natl. Acad. Sci. USA 85]: 2444. Alternatively, it may be determined using the Needleman-Wunsch algorithm (Needleman and Wunsch, 1970, J. Mol. Biol. 48: 443-453) as performed in the Needleman program of the EMBOSS package (EMBOSS: The European Molecular Biology Open Software Suite, Rice et al., 2000, Trends Genet. 16: 276-277) (version 5.0.0 or later) (comprising GCG program package ((Devereux, J., et al, Nucleic Acids Research 12: 387 (1984)), BLASTP, BLASTN, FASTA (Atschul, [S.] [F.,] [ET AL, J MOLEC BIOL 215]: 403 (1990); Guide to Huge Computers, Martin J. Bishop, [ED.,] Academic Press, San Diego,1994, and [CARILLO et al.](1988) SIAM J Applied Math 48: 1073). For example, BLAST of the National Center for Biotechnology Information or ClustalW may be used to determine homology, similarity, or identity.

The homology, similarity, or identity between polynucleotides or polypeptides may be determined by comparing sequence information using the GAP computer program as introduced by, for example, Needleman et al. (1970), J Mol Biol. 48:443, as disclosed by Smith and Waterman, Adv. Appl. Math (1981) 2:482. Briefly, the GAP program defines homology, similarity, or identity as the value obtained by dividing the number of similarly aligned symbols (i.e., nucleotides or amino acids) by the total number of the symbols in the shorter of the two sequences. The default parameters for the GAP program may comprise: (1) unitary matrices (comprising a value 1 for identity and a value 0 for non-identity), PAM Matrix (see those described by Schwartz and Dayhoff, eds., Atlas Of Protein Sequence And Structure, National Biomedical Research Foundation (1978)), and the weighted comparison matrix of Gribskov et al(1986) Nucl. Acids Res. 14: 6745 (or EDNAFULL (EMBOSS version of NCBI NUC4.4) substitution matrix); (2) a penalty of 3.0 for each gap and an additional 0.10 penalty for each symbol in each gap (or a gap open penalty of 10 and a gap extension penalty of 0.5); and (3) no penalty for end gaps.

Further, whether any two polynucleotide or polypeptide sequences have a homology, similarity or identity may be identified by comparing the sequences in a Southern hybridization experiment under stringent conditions as defined, and appropriate hybridization conditions defined are within the skill of the art, and may be determined by a method well known to those skilled in the art (e.g., J. Sambrook et al., Molecular Cloning, A Laboratory Manual; F.M. Ausubel et al., Current Protocols in Molecular Biology, John Wiley & Sons, Inc., New York), but are not limited thereto.

As used herein, the term "stringent conditions" means conditions that enable specific hybridization between polynucleotides. These conditions are specifically described in documents (see Sambrook et al., supra, 9.50-9.51, 11.7-11.8). Examples thereof comprise conditions in which polynucleotides having higher homology or identity, i.e., polynucleotides having 60% or more, 70% or more, 75% or more, 80% or more, 85% or more, 90% or more, 95% or more, 96% or more, 97% or more, 98% or more, or 99% or more homology or identity are hybridized with each other while polynucleotides having lower homology or identity are not hybridized with each other, or conditions in which washing is performed once, specifically, twice to three times at a salt concentration and temperature equivalent to 60°C, 1×SSC, 0.1% SDS, specifically 60°C, 0.1×SSC, 0.1% SDS, more specifically, 68°C, 0.1×SSC, 0.1% SDS, which are washing conditions for common Southern hybridization.

The hybridization requires that two nucleotides have complementary sequences, although mismatches between bases are allowed depending on the stringency of hybridization. The term "complementary" is used to describe the relation between nucleotide bases capable of being hybridized with each other. For example, with regard to DNA, adenine is complementary to thymine and cytosine is complementary to guanine. Hence, the polynucleotide of the present disclosure may also comprise substantially similar nucleotide sequences as well as isolated nucleic acid fragments that are complementary to the entire sequence.

For example, polynucleotides having homology or identity to the polynucleotide of the present disclosure may be detected using the hybridization conditions comprising a hybridization step at a Tm value of 55°C under the above-described conditions. Further, the Tm value may be 60°C, 63°C, or 65°C, but is not limited thereto, and may be appropriately adjusted by those skilled in the art depending on the purpose thereof.

The appropriate stringency to hybridize the polynucleotide depends on the length and degree of complementarity of the polynucleotide, and the variables are well known in the art (e.g., J. Sambrook et al., supra).

As used herein, the term "polyhydroxybutyrate (PHB)", which is a polymer of the polyhydroxyalkanoate series, is a polymer of 3-hydroxybutyrate, and is a compound belonging to polyester. The PHB may be used interchangeably with poly-3-hydroxybutyrate (P(3HB)), poly-3-hydroxybutanoate (P3HA), and a 3-hydroxybutyrate homopolymer.

As used herein, the term "methanotrophic bacterium" refers to a microorganism that is able to grow using methane as an energy source, and comprise all of wild-type methanotrophic bacteria, or methanotrophic bacteria in which genetic modification naturally or artificially occurs, and it may be a methanotrophic bacterium in which a specific mechanism is weakened or enhanced due to insertion of a foreign gene or an activity enhancement or inactivation of an endogenous gene, and it may be a methanotrophic bacterium comprising genetic modification for the production of a desired polypeptide, protein, or product. In the present disclosure, the terms "methanotrophic bacterium", "microorganism", and "strain" have the same meaning and may be used interchangeably without limitation.

For example, the methanotrophic bacterium of the present disclosure may be a methanotrophic bacterium (e.g., recombinant strain) in which the activity of malate thiokinase is enhanced, as compared to its endogenous activity, but is not limited thereto.

As used herein, the term "methanotrophic bacterium having the PHB-producing ability" is a methanotrophic bacterial strain capable of producing PHB within a living organism, and may comprise both a methanotrophic bacterium prepared by providing the PHB-producing ability to a parent strain without the PHB-producing ability, or a methanotrophic bacterium inherently having the PHB-producing ability. The PHB-producing ability may be conferred or enhanced by species improvement.

As used herein, the term "unmodified methanotrophic bacterium" does not exclude strains comprising a mutation that may occur naturally in methanotrophic bacteria, and may refer to a wild-type strain or a natural strain itself or may be a strain before the trait is changed by genetic variation due to natural or artificial factors. The term "unmodified methanotrophic bacterium" may be used interchangeably with a "strain before being modified", "methanotrophic bacterium before being modified", "unvaried strain", "unmodified strain", "unvaried methanotrophic bacterium", "parent strain before being modified", "wild-type methanotrophic bacterium", "reference methanotrophic bacterium", or "standard methanotrophic bacterium". In the present disclosure, the unmodified methanotrophic bacterium may refer to a strain in which the activity of malate thiokinase of the present disclosure is not enhanced or has not yet been enhanced, as compared to its endogenous activity, but is not limited thereto. In addition, in the present disclosure, the unmodified methanotrophic bacterium may be a methanotrophic bacterium comprising the amino acid sequence(s) consisting of SEQ ID NO: 1 and/or SEQ ID NO: 2, or the polynucleotide(s) consisting of SEQ ID NO: 3 and/or SEQ ID NO: 4, but is not limited thereto.

For example, the methanotrophic bacterium of the present disclosure may comprise any methanotrophic bacterium capable of producing the desired PHB by enhancing the activity of malate thiokinase, as compared to its endogenous activity. For example, the methanotrophic bacterium of the present disclosure is characterized in that the PHB-producing ability is increased by enhancing the activity of malate thiokinase, as compared to its endogenous activity, and may be a genetically modified methanotrophic bacterium or recombinant methanotrophic bacterium, but is not limited thereto. Specifically, the recombinant strain in which the PHB-producing ability is increased may be a natural wild-type methanotrophic bacterium or a methanotrophic bacterium in which the PHB-producing ability is increased, as compared to the unmodified methanotrophic bacterium having the endogenous activity of malate thiokinase, but is not limited thereto.

For example, the "methanotrophic bacterium having the PHB-producing ability" refers to a methanotrophic bacterial strain capable of producing PHB within a living organism, and may comprise both a methanotrophic bacterium inherently having the PHB-producing ability or a methanotrophic bacterium prepared by providing the PHB-producing ability to a parent strain without the PHB-producing ability due to the enhanced activity of malate thiokinase of the present disclosure. The PHB-producing ability may be conferred or enhanced by species improvement.

For example, the recombinant methanotrophic bacterium having the PHB-producing ability of the present disclosure may comprise any methanotrophic bacterium capable of producing PHB, in which the activity of malate thiokinase of the present disclosure is enhanced by transformation with a vector.

As used herein, the term "enhancement (increase)" in the activity of the polypeptide means that the activity of the polypeptide is enhanced, as compared to its endogenous activity. The enhancement may be used interchangeably with the term activation, up-regulation, overexpression, increase (enhancement), etc.

The enhancement may comprise both cases of exhibiting the activity not originally possessed, or exhibiting the improved activity, as compared to the endogenous activity or the activity before modification.

For example, "exhibiting the activity not originally possessed" may be "introduction of a protein", but is not limited thereto. The introduction of the protein means that a gene that is not originally possessed by the methanotrophic bacterium is expressed within the methanotrophic bacterium to exhibit the activity of a specific protein or to exhibit the enhanced or improved activity as compared to the endogenous activity of the corresponding protein or the activity before modification. For example, a polynucleotide encoding a specific protein may be introduced into a chromosome in the methanotrophic bacterium, or a vector comprising a polynucleotide encoding the specific protein may be introduced into the methanotrophic bacterium, thereby exhibiting its activity.

The term "endogenous activity" means the activity of a specific polypeptide originally possessed by a parent strain before the trait is changed or an unmodified methanotrophic bacterium, when the trait is changed by genetic variation due to natural or artificial factors. This may be used interchangeably with "activity before modification".

The fact that the activity of a polypeptide is enhanced as compared to the endogenous activity means that the activity of the polypeptide is improved as compared to the activity and/or concentration (expression level) of the specific polypeptide originally possessed by a parent strain before the trait is changed or an unmodified methanotrophic bacterium.

For example, the enhancement indicates that the activity of the corresponding protein, which was originally absent, appears, or its activity or concentration is generally increased by about 1%, about 10%, about 25%, about 50%, about 75%, about 100%, about 150%, about 200%, about 300%, about 400% or about 500%, and in some cases by at least about 1000% or about 2000% or even more, based on the activity or concentration in the wild-type protein or the initial methanotrophic bacterial strain, but is not limited thereto.

The enhancement of the activity of the polypeptide may be achieved through the introduction of a foreign polypeptide or the enhancement of the activity of the endogenous polypeptide. The enhancement of the activity of the polypeptide may be confirmed by enhancement in the degree of activity and the expression level of the corresponding polypeptide or enhancement in the amount of the product released from the corresponding polypeptide.

For the enhancement of the activity of the polypeptide, various methods well known in the art may be applied, and the method is not limited as long as the activity of the desired polypeptide may be enhanced as compared to that of the methanotrophic bacterium before modification. Specifically, genetic engineering and/or protein engineering well known to those skilled in the art, which are routine methods of molecular biology, may be used, but the method is not limited thereto (e.g., Sitnicka et al. Functional Analysis of Genes. Advances in Cell Biology. 2010, Vol. 2. 1-16, Sambrook et al. Molecular Cloning 2012, etc.).

Specifically, the enhancement of the activity of the polypeptide of the present disclosure may be:
1) enhancement in the intracellular copy number of the polynucleotide encoding the polypeptide;
2) modification of a gene expression regulatory region on a chromosome encoding the polypeptide (e.g., occurrence of variations in the expression regulatory region, replacement with a sequence having stronger activity, or insertion of a sequence having stronger activity);
3) modification of a nucleotide sequence encoding a start codon or 5'-UTR region of the gene transcript encoding the polypeptide;
4) modification of the amino acid sequence of the polypeptide to enhance the activity of the polypeptide;
5) modification of the polynucleotide sequence encoding the polypeptide to enhance the activity of the polypeptide (e.g., modification of the polynucleotide sequence of the polypeptide gene to encode the polypeptide that has been modified to enhance the activity of the polypeptide);
6) introduction of a foreign polypeptide exhibiting the activity of the polypeptide or a foreign polynucleotide encoding the same;
7) codon optimization of the polynucleotide encoding the polypeptide;
8) analysis of the tertiary structure of the polypeptide to select and to modify or chemically modify the exposed site;
9) controlling of intracellular localization of the polypeptide; or
10) a combination of two or more selected from 1) to 9), but is not particularly limited thereto.

For example, 1) the enhancement in the intracellular copy number of the polynucleotide encoding the polypeptide may be achieved by the introduction, into a host cell, of a vector to which the polynucleotide encoding the corresponding polypeptide is operably linked and which is able to replicate and function independently of the host. Alternatively, the enhancement may be achieved by the introduction of one or two or more copies of the polynucleotide encoding the corresponding polypeptide into the chromosome in a host cell. The introduction into the chromosome may be performed by the introduction, into a host cell, of a vector capable of inserting the polynucleotide into the chromosome in the host cell, but is not limited thereto.

2) The replacement of a gene expression regulatory region (expression regulatory sequence) on the chromosome encoding the polypeptide with a sequence with strong activity may be, for example, the occurrence of variations in the sequence through deletion, insertion, non-conservative or conservative substitution, or a combination thereof, or the replacement with a sequence having stronger activity, to further enhance the activity of the expression regulatory region. The expression regulatory region may comprise, but is not particularly limited to, a promoter, an operator sequence, a sequence encoding a ribosome binding site, a sequence controlling the termination of transcription and translation, etc. For example, it may be the replacement of the original promoter with a stronger promoter, but is not limited thereto.

Examples of the known stronger promoter may comprise cj1 to cj7 promoters (US Patent No. 7662943 B2), lac promoter, trp promoter, trc promoter, tac promoter, lambda phage PR promoter, PL promoter, tet promoter, gapA promoter, SPL7 promoter, SPL13 (sm3) promoter (US Patent No. 10584338 B2), O2 promoter (US Patent No. 10273491 B2), tkt promoter, yccA promoter, etc., but are not limited thereto.

3) The modification of a nucleotide sequence encoding a start codon or 5'-UTR region of the gene encoding the polypeptide may be, for example, replacing with another start codon having a higher polypeptide expression rate than the endogenous start codon, but is not limited thereto.

4) and 5) The modification of the amino acid sequence or polynucleotide sequence may be the occurrence of variations in the sequence through deletion, insertion, non-conservative or conservative substitution, or a combination thereof in the amino acid sequence of the polypeptide or the polynucleotide sequence encoding the polypeptide, or the replacement with an amino acid sequence or polynucleotide sequence improved to have stronger activity or an amino acid sequence or polynucleotide sequence improved to have enhanced activity, in order to enhance activity of the polypeptide, but is not limited thereto. Specifically, the replacement may be performed by inserting the polynucleotide into the chromosome by homologous recombination, but is not limited thereto. The vector used herein may further comprise a selection marker for checking chromosome insertion. The selection marker is as described above.

6) The introduction of a foreign polynucleotide exhibiting the activity of the polypeptide may be the introduction, into a host cell, of a foreign polynucleotide encoding a polypeptide exhibiting the identical/similar activity to the polypeptide. The foreign polynucleotide is not limited to the origin or sequence thereof as long as it exhibits the identical/similar activity to the polypeptide. The method used in the introduction may be performed by any known transformation method appropriately selected by a person skilled in the art, and through the expression of the introduced polynucleotide in the host cell, the polypeptide is produced and the activity thereof may be enhanced.

7) The codon optimization of the polynucleotide encoding the polypeptide may be the codon optimization of an endogenous polynucleotide to enhance transcription or translation thereof in a host cell, or the codon optimization of a foreign polynucleotide to allow optimized transcription or translation thereof in a host cell.

8) The modification or chemical modification of an exposed site selected by analysis of a tertiary structure of the polypeptide may be, for example, the modification or chemical modification of an exposed site to be modified or chemically modified by comparing sequence information of a polypeptide to be analyzed with a database that stores sequence information of known proteins, determining a template protein candidate according to similarity of the sequences, and identifying the structure based thereon.

9) The controlling of intracellular localization of the polypeptide may mean targeting the polypeptide to a specific organelle within cells or to a specific intracellular space, for example, targeting to the periplasm or cytoplasm through the addition or removal of a leader sequence that functions in targeting the polypeptide, but is not limited thereto.

Such enhancement of the activity of the polypeptide may mean that the activity or concentration of the corresponding polypeptide is enhanced relative to the activity or concentration of the polypeptide expressed in the wild-type or the methanotrophic bacterial strain before modification, or that the amount of a product produced from the corresponding polypeptide is increased, but is not limited thereto.

For example, the recombinant methanotrophic bacterium having the PHB-producing ability of the present disclosure may comprise any methanotrophic bacterium that is transformed with a vector to have the enhanced activity of malate thiokinase of the present disclosure, thereby producing PHB.

For example, in the methanotrophic bacterium of the present disclosure, the enhancement may be, but is not limited to, enhancement of the intracellular copy number; modification of a gene expression regulatory region; modification of a nucleotide sequence encoding a start codon or 5'-UTR region; modification of the amino acid sequence of the polypeptide; modification of the polynucleotide sequence; introduction of a foreign polypeptide or a foreign polynucleotide encoding the same; codon optimization of the polynucleotide; modification of the structure of the polypeptide; or a combination thereof.

For example, the enhancement of the intracellular copy number of the polynucleotide encoding the polypeptide in the methanotrophic bacterium of the present disclosure may be the increase in the copy number by inserting the polynucleotide into an expression vector and introducing the expression vector into a host cell. The polynucleotide may comprise an intergenic region, but is not limited thereto.

For example, the intergenic region of mtkA and mtkB genes each encoding MtkA and MtkB proteins may be a polynucleotide having or comprising a nucleotide sequence of SEQ ID NO: 5, or consisting of or essentially consisting of the nucleotide sequence, but is not limited thereto.

For example, the mtkAB operon, which represents the entire operon encompassing all of the mtkAB genes, by comprising the intergenic region of the mtkA and mtkB genes, may comprise a nucleotide sequence of SEQ ID NO: 6, but is not limited thereto.

The vector of the present disclosure may comprise a DNA construct comprising a nucleotide sequence of a polynucleotide encoding a target polypeptide which is operably linked to a suitable expression regulatory region (or expression regulatory sequence) so that the target polypeptide may be expressed in a suitable host. The expression regulatory region may comprise a promoter capable of initiating transcription, any operator sequence for controlling the transcription, a sequence encoding a suitable mRNA ribosome binding site, and a sequence controlling termination of transcription and translation. The vector may be transformed into a suitable host cell, and then replicated or function independently of the host genome, or may be integrated into the genome itself.

The vector used in the present disclosure is not particularly limited, but any vector known in the art may be used. Examples of commonly used vectors may comprise natural or recombinant plasmids, cosmids, viruses, and bacteriophages. For example, pWE15, M13, MBL3, MBL4, IXII, ASHII, APII, t10, t11, Charon4A, and Charon21A, etc. may be used as a phage vector or a cosmid vector, and pDZ system, pBR system, pUC system, pBluescript II system, pGEM system, pTZ system, pCL system, pSK system, pSKH system, and pET system, etc. may be used as a plasmid vector. Specifically, a pDZ, pDC, pDCM2, pACYC177, pACYC184, pCL, pSK, pSKH130, pECCG117, pUC19, pBR322, pMW118, pCC1BAC vector, etc. may be used.

For example, a polynucleotide encoding a target polypeptide may be inserted into a chromosome through a vector for intracellular chromosome insertion. Insertion of the polynucleotide into the chromosome may be performed by any method known in the art, for example, homologous recombination, but is not limited thereto. The vector may further comprise a selection marker for checking chromosome insertion. The selection marker is for selecting the cells transformed with vectors, i.e., for checking the insertion of a target nucleic acid molecule, and markers that confer selectable phenotypes such as drug resistance, auxotrophy, resistance to cytotoxic agents, or expression of surface polypeptides may be used. In an environment treated with a selective agent, only cells expressing the selection marker survive or exhibit other phenotypic traits, and thus transformed cells may be selected.

As used herein, the term "transformation" means that a vector comprising a polynucleotide encoding a target polypeptide is introduced into a host cell or methanotrophic bacterium so that the polypeptide encoded by the polynucleotide is expressed in the host cell. As long as the transformed polynucleotide may be expressed in the host cell, it may be integrated into and located in the chromosome of the host cell or exist extrachromosomally, or it may be in any of the cases. Further, the polynucleotide comprises DNA and/or RNA encoding the target polypeptide. The polynucleotide may be introduced in any form as long as it may be introduced into a host cell and expressed therein. For example, the polynucleotide may be introduced into a host cell in the form of an expression cassette, which is a gene construct comprising all elements required for self-expression. The expression cassette may usually comprise a promoter operably linked to the polynucleotide, a transcription termination signal, a ribosome binding site, and a translation termination signal. The expression cassette may be in the form of an expression vector capable of self-replicating. Further, the polynucleotide may be introduced into a host cell in its own form and operably linked to a sequence required for expression in the host cell, but is not limited thereto.

As used herein, the term "operably linked" refers to a configuration of placing a regulatory sequence in an appropriate position to direct expression of a coding sequence. Thus, the term "operably linked" comprises an attachment or linking between a regulatory region of a functional domain having a known or desired activity such as a promoter, a terminator, a signal sequence, or an enhancer region, and a target (gene or polypeptide) such that the expression, secretion, or function of the target (gene or polypeptide) may be regulated in accordance with the known or desired activity. For example, the term means that the polynucleotide sequence is functionally linked to a promoter sequence that initiates and mediates transcription of the polynucleotide encoding the target variant polypeptide of the present disclosure.

As used herein, the term "expression" comprises any step involved in production of a polypeptide, e.g., transcription, post-transcriptional modification, translation, post-translational modification, and secretion, but is not limited thereto.

As used herein, the term "expression vector" refers to a linear or circular nucleic acid molecule comprising a coding sequence and an operably linked regulatory sequence for expression thereof.

As used herein, the term "regulatory sequence" refers to a polynucleotide sequence required for the expression of a coding sequence. Each regulatory sequence may be native (derived from the same origin) or foreign (derived from different genes) to the coding sequence. Examples of the regulatory sequence may comprise a leader sequence, a polyadenylation sequence, a propeptide sequence, a promoter, a signal peptide sequence, an operator sequence, a ribosome binding site-encoding sequence, and a sequence terminating transcription and translation. A minimum unit of the regulatory sequence may comprise a promoter and a sequence terminating transcription and translation.

With regard to a cell, polynucleotide, polypeptide, or vector, the term "recombinant" used herein refers to the cell, polynucleotide, polypeptide, or vector modified by introduction of a heterologous nucleic acid or polypeptide or alteration of a native polynucleotide or polypeptide, or a cell derived from the modified cell. Thus, for example, recombinant cells may express genes that are not found within the native (non-recombinant) form of the cells, or may express native genes that are otherwise abnormally expressed, underexpressed or not expressed at all.

For example, the methanotrophic bacterium producing PHB may be a methanotrophic bacterium inherently comprising the protein(s) consisting of the amino acid sequence(s) of SEQ ID NO: 1 and/or SEQ ID NO: 2, or a protein(s) consisting of an amino acid sequence(s) having at least 60%, 70%, 75%, 80%, 85%, 90%, 95%, 96%, 97%, 98%, 99%, 99.5%, 99.7% or 99.9% or more homology or identity to SEQ ID NO: 1 and/or SEQ ID NO: 2.

For example, the methanotrophic bacterium producing PHB may be a methanotrophic bacterium inherently comprising a polynucleotide sequence(s) capable of encoding a protein(s) comprising an amino acid sequence(s) having at least 80% homology to SEQ ID NO: 1 and/or SEQ ID NO: 2, the nucleotide sequence(s) of SEQ ID NO: 3 and/or SEQ ID NO: 4, or a nucleotide sequence(s) having 60% or more, 70% or more, 75% or more, 80% or more, 85% or more, 90% or more, 95% or more, 96% or more, 97% or more, 98% or more, and less than 100% homology or identity to the nucleotide sequence(s) of SEQ ID NO: 3 and/or SEQ ID NO: 4.

The methanotrophic bacterium of the present disclosure may comprise any methanotrophic bacterium in which the activity of malate thiokinase is enhanced as compared to its endogenous activity by various known methods.

For example, the methanotrophic bacterium with the increased PHB-producing ability of the present disclosure may be a methanotrophic bacterium, in which the PHB-producing ability is increased, as compared to an unmodified methanotrophic bacterium, but is not limited thereto. For example, the unmodified methanotrophic bacterium which is a subject strain for comparing whether or not the PHB-producing ability is increased may be an OB3b strain of the genus *Methylocystis,* but is not limited thereto.

For example, the methanotrophic bacterium with the increased PHB-producing ability may have an increased PHB-producing ability of about 1% or more, specifically, about 1% or more, about 2.5% or more, about 5% or more, about 6% or more, about 7% or more, about 8% or more, about 9% or more, about 10% or more, about 15% or more, about 20% or more, about 25%or more, or about 30% or more (the upper limit is not particularly limited, but may be, for example, about 200% or less, about 150% or less, about 100% or less, about 50% or less, about 45% or less, or about 40% or less), as compared to the PHB-producing ability of the parent strain before modification or the unmodified methanotrophic bacterium, but the increased amount is not limited thereto as long as the production ability has an increased amount of a + value, as compared to the production ability of the parent strain before modification or the unmodified methanotrophic bacterium. In another example, the recombinant strain with the increased PHB-producing ability may have an increased PHB-producing ability of about 1.1 times or more, about 1.15 times or more, about 1.2 times or more, about 1.25 times or more, or about 1.3 times or more (the upper limit is not particularly limited, but may be, for example, about 10 times or less, about 5 times or less, about 3 times or less, about 2 times or less, about 1.5 times or less, or about 1.4 times or less), as compared to the parent strain before modification or the unmodified methanotrophic bacterium, but is not limited thereto.

For still another example of the present disclosure, the methanotrophic bacterium having the PHB-producing ability may comprise, for example, methanotrophic bacterial strains of the genus *Methylocystis,* the genus *Methylomonas,* the genus *Methylobacter,* the genus *Methylococcus,* the genus *Methylomicrobium,* the genus *Methylosphaera,* the genus *Methylocaldum,* the genus *Methyloglobus,* the genus *Methylosarcina,* the genus *Methyloprofundus,* the genus *Methylothermus,* the genus *Methylohalobius,* the genus *Methylogaea,* the genus *Methylomarinum,* the genus *Methylovulum,* the genus *Methylomarinovum,* the genus *Methylorubrum,* the genus *Methyloparacoccus,* the genus *Methylosinus,* the genus *Methylocella,* the genus *Methylocapsa,* the genus *Methylofurula,* the genus *Methylacidiphilum,* the genus *Methylacidimicrobium.*

With regard to the methanotrophic bacterium according to any one of the aforementioned specific embodiments, the methanotrophic bacterium of the present disclosure may be a microorganism of the genus *Methylocystis.* Specifically, the methanotrophic bacterium of the present disclosure may be an OB3b strain of the genus *Methylocystis,* but is not limited thereto.

Another aspect of the present disclosure provides a method of producing PHB, the method comprising the step of culturing, in a medium, the methanotrophic bacterium having the PHB-producing ability, in which the activity of malate thiokinase is enhanced, as compared to its endogenous activity.

As used herein, the term "culturing" refers to growing the strain of the present disclosure under appropriately adjusted environmental conditions. The culture procedure of the present disclosure may be performed according to appropriate media or culture conditions known in the art. Such a culture procedure may be easily adjusted according to the selected strain by a person skilled in the art. Specifically, the culturing may be in a batch type, a continuous type, and/or a fed-batch type, but is not limited thereto.

As used herein, the term "medium" refers to a mixture comprising, as main ingredients, nutrient materials required for culturing the methanotrophic bacterium of the present disclosure, wherein the medium supplies nutrient materials comprising water essential for survival and growth, growth factors, etc. Specifically, as for the media and other culture conditions used for culturing the strain of the present disclosure, any medium that is used for usual culture of methanotrophic bacteria may be used without particular limitation. However, the methanotrophic bacterium of the present disclosure may be cultured under aerobic conditions in a common medium comprising appropriate carbon sources, nitrogen sources, phosphorus sources, inorganic compounds, amino acids, and/or vitamins, while controlling the temperature, pH, etc.

In the present disclosure, as for the carbon source, methanol may be mainly used due to the nature of methanotrophic bacteria, and additionally, the carbon source may comprise carbohydrates, such as glucose, saccharose, lactose, fructose, sucrose, maltose, etc.; sugar alcohols, such as mannitol, sorbitol, etc.; organic acids, such as pyruvic acid, lactic acid, citric acid, etc.; and amino acids, such as glutamic acid, methionine, lysine, etc. In addition, natural organic nutrient sources may be used, such as starch hydrolysates, molasses, blackstrap molasses, rice bran, cassava, bagasse, and corn steep liquor, and specifically, carbohydrates, such as glucose and sterile pretreated molasses (i.e., molasses converted to reduced sugars) may be used, and appropriate amounts of various other carbon sources may be used without limitation. These carbon sources may be used alone or in a combination of two or more thereof, but are not limited thereto.

As for the nitrogen sources, inorganic nitrogen sources, such as ammonia, ammonium sulfate, ammonium chloride, ammonium acetate, ammonium phosphate, ammonium carbonate, ammonium nitrate, etc.; amino acids, such as glutamic acid, methionine, glutamine, etc.; and organic nitrogen sources, such as peptone, NZ-amine, meat extracts, yeast extracts, malt extracts, corn steep liquor, casein hydrolysates, fish or decomposition products thereof, defatted soybean cake or decomposition products thereof, etc. may be used. These nitrogen sources may be used alone or in a combination of two or more thereof, but are not limited thereto.

The phosphate sources may comprise potassium phosphate monobasic, potassium phosphate dibasic, and sodium-comprising salts corresponding thereto. As for inorganic compounds, sodium chloride, calcium chloride, iron chloride, magnesium sulfate, iron sulfate, manganese sulfate, calcium carbonate, etc. may be used, and in addition, amino acids, vitamins, and/or suitable precursors may be comprised. These components or precursors may be added to the medium in a batch or continuous manner. However, the present disclosure is not limited thereto.

Further, the pH of the medium may be adjusted by adding compounds, such as ammonium hydroxide, potassium hydroxide, ammonia, phosphoric acid, sulfuric acid, etc., to the medium in an appropriate manner during the culture of the methanotrophic bacterium of the present disclosure. In addition, an anti-foaming agent, such as a fatty acid polyglycol ester, may be used to suppress foam formation during the culture. In addition, oxygen or oxygen-comprising gas may be injected into the medium to maintain the aerobic state of the medium, or no gas may be injected, or nitrogen, hydrogen, or carbon dioxide gas may be injected to maintain the anaerobic or non-aerobic state, but is not limited thereto.

In the culture of the present disclosure, the culture temperature may be maintained at 27°C to 37°C, specifically at 30°C to 33°C, and the culture may be performed for about 20 hours to 120 hours, but is not limited thereto.

As used herein, the term "culture" refers to a culture liquid obtained by culturing a specific methanotrophic bacterium in a culture medium, a concentrated culture liquid, a dry product of the culture liquid, a culture filtrate, a concentrated culture filtrate, or a dry product of the culture filtrate, and the culture liquid refers to those comprising the specific methanotrophic bacterium, and the culture filtrate refers to those substantially not comprising the specific methanotrophic bacterium (which means that the methanotrophic bacterium to be separated by filtration, etc. is substantially excluded, which does not mean that the methanotrophic bacterium is completely excluded from the filtrate.). The formulation of the culture is not limited, but it may be, for example, a liquid, an emulsion, or a solid. Specifically, with respect to the objects of the present disclosure, the culture may comprise PHB.

As used herein, the term "fermentation" refers to a process whereby methanotrophic bacteria break down organic materials using their own enzymes, excluding putrefaction. Fermentation and putrefaction proceed by similar processes. However, when useful substances are produced as a result of decomposition, it is called fermentation, and when bad smells or harmful substances are produced, it is called putrefaction.

In the present disclosure, the method of obtaining the fermentation product from the strain is not particularly limited, and the fermentation product may be obtained according to a method commonly used in the art or similar art.

As used herein, the term "fermentation product" comprises not only a fermented material itself, but also all types of materials comprising the fermentation product generated from the strain, such as a culture medium of a strain in which the strain and the culture coexist, a fermentation product obtained by filtering the strain from the culture medium, a fermentation product obtained by sterilizing the strain from the culture medium and filtering the same, an extract obtained by extracting the fermentation product or the culture medium comprising the same, a dilution obtained by diluting the fermentation product or the extract thereof, a concentrate, a dry product obtained by drying the fermentation product or the extract thereof, a lysate obtained by collecting and disrupting cells of the strain, etc.

In the method of the present disclosure, the culturing of the methanotrophic bacterium may be performed using any culturing conditions and culturing method known in the art. Such culturing procedure may be easily adjusted for use by a person skilled in the art according to the selected strain.

PHB produced by culturing of the present disclosure may be released into the medium or may remain in cells.

In one specific embodiment, the method of producing PHB of the present disclosure may further comprise the steps of preparing the methanotrophic bacterium of the present disclosure, preparing a medium for culturing the strain, or a combination of these steps (regardless of the order, in any order), for example, before the culturing step.

The method of producing PHB of the present disclosure may further comprise the step of recovering a desired substance, specifically, PHB, from the cultured methanotrophic bacterium, a culture of the methanotrophic bacterium, a fermentation product of the methanotrophic bacterium, or the culture medium. The recovering step may be further comprised after the culturing step.

The recovering may be collecting the desired PHB by using an appropriate method known in the art according to the method of culturing the methanotrophic bacterium of the present disclosure, for example, a batch, continuous, or fed-batch type culture. For example, centrifugation, filtration, treatment with a crystallized protein precipitating agent (salting-out), extraction, sonication, ultrafiltration, dialysis, various types of chromatography, such as molecular sieve chromatography (gel filtration), adsorption chromatography, ion exchange chromatography, affinity chromatography, HPLC, and a combination of these methods may be used, and a desired substance, specifically, PHB may be recovered from the medium or methanotrophic bacterium by using an appropriate method known in the art.

In addition, the method of producing PHB of the present disclosure may further comprise a purification step. The purification may be performed by using an appropriate method known in the art. In an exemplary embodiment, when the method of producing PHB of the present disclosure comprises both the recovering step and the purification step, the recovering step and the purification step may be performed continuously or discontinuously regardless of the order, or may be performed simultaneously or integrated into one step, but is not limited thereto.

With regard to the method of the present disclosure, the enhancement of the activity of malate thiokinase and PHB, etc. are as described in other aspects.

Still another aspect of the present disclosure provides a composition for producing PHB, the composition comprising the methanotrophic bacterium having the PHB-producing ability of the present disclosure, in which the activity of malate thiokinase is enhanced, as compared to its endogenous activity, a culture of the methanotrophic bacterium, a fermentation product of the methanotrophic bacterium, or a combination of two or more thereof.

The composition of the present disclosure may further comprise any appropriate excipient that is usually used in the composition for producing PHB, and examples of the excipient may comprise a preserving agent, a wetting agent, a dispersing agent, a suspending agent, a buffer, a stabilizing agent, an isotonic agent, etc., but are not limited thereto.

In a specific embodiment, each component present in the composition of the present disclosure may be comprised in a microbially effective amount, or in an amount that may be appropriately present in the composition for production.

With regard to the composition of the present disclosure, the enhancement of the activity of malate thiokinase and PHB, etc. are as described in other aspects.

Still another aspect of the present disclosure provides use of the methanotrophic bacterium of the present disclosure, in which the activity of malate thiokinase is enhanced, as compared to its endogenous activity, in producing PHB.

With regard to the use of the present disclosure, the enhancement of the activity of malate thiokinase and PHB, etc. are as described in other aspects.

### [Mode for Carrying Out the Invention]

Hereinafter, the present disclosure will be described in more detail by way of exemplary embodiments. However, the following exemplary embodiments are only preferred embodiments for illustrating the present disclosure, and thus are not intended to limit the scope of the present disclosure thereto. Meanwhile, technical matters not described in the present specification may be sufficiently understood and easily implemented by those skilled in the technical field of the present disclosure or similar technical fields.

### Example 1. Preparation of a strain with enhanced MtkAB expression and Evaluation of PHB-producing ability

### Example 1-1. Construction of a recombinant vector with mktAB-gene

To construct plasmid containing an mtkAB gene(mtkA; SEQ ID NO: 1, mtkB; SEQ ID NO: 2), a DNA fragment amplified through PCR using pAWP89 (Appl Environ Microbiol. 2015 Mar;81(5):1775-1781) as a template and vec_F and vec_R primers, a DNA fragment amplified through PCR using DNA (SEQ ID NO: 6) synthesized from mtkAB derived from *Methylosinus* sp. C49 (NCBI Taxonomy ID: 2699395) as a template and mtkA_F and mtkB_R primers, and a DNA fragment amplified through PCR using genomic DNA of *Escherichia coli* K-12 as a template and TrrnB_F and TrrnB_R primers were cloned using the In-Fusion HD Cloning Kit (Takara, Cat. No. 639650). The final completed plasmid had kanamycin resistance, and was named pME-01.

The sequences of the primers used for vector construction are as follows.

**[Table 1]**

| SEQ ID NO. | Name | Sequence |
|---|---|---|
| 7 | TrrnB_F | CGTCAAAAGGGCGACACCCCCT AATTAGCCCGGGCGAAAGGCCC |
| 8 | TrrnB_R | CGCCCCTACGGCGCCCACTGCTCTAGAGGATCCCCTTTGC C TGGCGGCAGTAGCG |
| 9 | mtkA_F | TTCACACAGGAAACAGCT ATGGACGTCCATGAGTATCAAGC |
| 10 | mtkB_R | GGGGTGTCGCCCTTTTGACG TCACGCGCGCTTCAGCACG |
| 11 | Vec_F | CAGTGGGCGCCGTAGGGGCG GCGTAATGCTCTGCCAGTG |
| 12 | Vec_R | CATAGCTGTTTCCTGTGTGAATAC |

### Example 1-2. Preparation of PHB-producing strain with enhanced mktAB gene expression

An electroporation method was used to introduce the pME-01 vector constructed in Example 1-1 into *Methylosinus trichosporium* OB3b (KCTC12760) strain. The strain containing the introduced plasmid was selected on an NMS solid medium supplemented 5 µg/mL kanamycin. The OB3b straincontaining pAWP89 was as OB3b-C as a control group, while OB3b straincontaining pME-01 was designated as Ob3b-M as an experimental group.

The medium used for culturing OB3b was NMS (Biotechnology for Biofuels volume 12, 234 (2019)), and 5 µM of CuSO₄ was supplied. For solid culture, 30% of the air was removed using a vacuum desiccator, and then supplemented with gas in which methane and carbon dioxide were mixed at a ratio of 9:1. A sealed flask for liquid culture was used to supply a culture solution at a level of 3% to 10% of the flask volume, and after sufficiently replacing the internal air with gas in which air:methane:carbon dioxide were mixed at a ratio of 70:30:3, the culture was performed at 270 rpm. The culture temperature was 35°C.

### Example 1-3. Evaluation of PHB-producing ability of strain with enhanced mktAB gene expression

To measure the PHB-producing ability of the OB3b-C and OB3b-M strains prepared in Example 1-2, PHB production was examined using an NMS2 medium, which was prepared by adjusting the KNO₃ concentration to 5 mM in an NMS medium.

The initial inoculation concentration was OD 0.2, and the gas in the flask was replaced twice a day after 24 hours from the start of culture, and cultured for a total of 72 hours. Finally, 15 mL of culture solution was harvested to obtain cells, and then freeze-dried to measure the dry cell weight, and PHB was analyzed by gas chromatography to examine the intracellular PHB content.

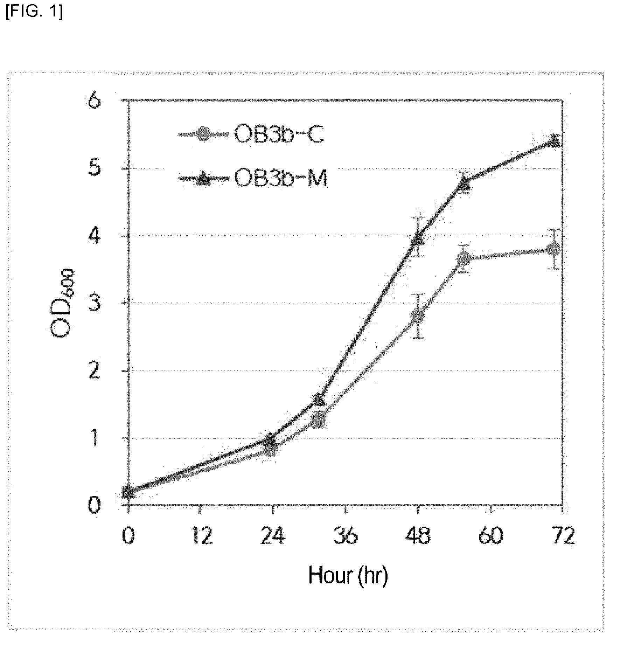

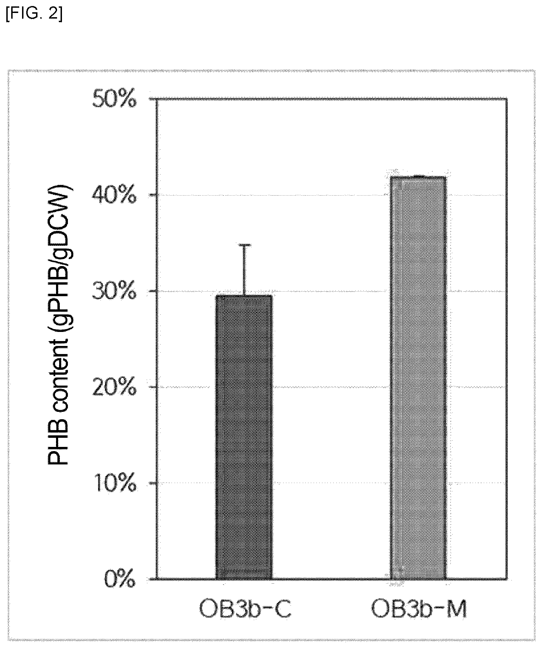

In detail, in order to examine cell growth, 0.2 mL was sampled at the time of gas replacement, and absorbance at 600 nm was measured to determine the cell concentration (FIG. 1), and the final culture solution was used to perform the PHB content analysis (FIG. 2 and Table 2).

**[Table 2]**

| Name of strain | Dry cell weight (mg) | PHB (mg) | PHB content (%) |
|---|---|---|---|
| OB3b-C | 17.9 | 5.3 | 29.5% |
| OB3b-M | 22.7 | 9.5 | 41.9% |

As shown in FIG. 1, it was confirmed that OB3b-M showed a slightly higher OD600 value than the control group OB3b-C after 30 hours.

As shown in Table 2 and FIG. 2, it was confirmed that the PHB content in the OB3b-M strain was 41.9%, indicating about a 1.4 times increase, as compared to 29.5% of the control group strain (OB3b-C). In other words, it was confirmed that when the expression of the mtkAB gene is enhanced in the methanotrophic bacterium, the PHB-producing ability may be increased while maintaining the growth ability of the methanotrophic bacterium.

Based on the above description, it will be understood by those skilled in the art that the present disclosure may be implemented in a different specific form without changing the technical spirit or essential characteristics thereof. In this regard, it should be understood that the above embodiment is not limitative, but illustrative in all aspects. The scope of the disclosure is defined by the appended claims rather than by the description preceding them, and therefore all changes and modifications that fall within metes and bounds of the claims, or equivalents of such metes and bounds are therefore intended to be embraced by the claims.

## Claims

1. A methanotrophic bacterium having a polyhydroxybutyrate (PHB)-producing ability, wherein the activity of malate thiokinase is enhanced, as compared to its endogenous activity.

2. The methanotrophic bacterium of claim 1, wherein the enhancement is enhancement of the intracellular copy number; modification of a gene expression regulatory region; modification of a nucleotide sequence encoding a start codon or 5'-UTR region; modification of an amino acid sequence of a polypeptide; modification of a polynucleotide sequence; introduction of a foreign polypeptide or a foreign polynucleotide encoding the foreign polypeptide; codon optimization of a polynucleotide; structural modification of a polypeptide; or a combination thereof.

3. The methanotrophic bacterium of claim 1, wherein the malate thiokinase is any one or more proteins selected from the group consisting of MtkA and MtkB.

4. The methanotrophic bacterium of claim 3, wherein the MtkA protein consists of an amino acid sequence of SEQ **ID** NO: 1, and the MtkB protein consists of an amino acid sequence of SEQ ID NO: 2.

5. The methanotrophic bacterium of claim 1, wherein the methanotrophic bacterium is a methanotrophic bacterium of the genus *Methylocystis (Methylocystis* sp.).

6. The methanotrophic bacterium of any one of claims 1 to 5, wherein the methanotrophic bacterium has an increased PHB-producing ability, as compared to an unmodified methanotrophic bacterium.

7. A method of producing PHB, the method comprising the step of culturing, in a medium, a methanotrophic bacterium having a PHB-producing ability, wherein the activity of malate thiokinase is enhanced, as compared to its endogenous activity.

8. The method of claim 7, further comprising the step of recovering PHB from the cultured methanotrophic bacterium, a culture of the methanotrophic bacterium, a fermentation product of the methanotrophic bacterium, or the culture medium.

9. A composition for producing PHB, the composition comprising a methanotrophic bacterium having a PHB-producing ability, wherein the activity of malate thiokinase is enhanced, as compared to its endogenous activity, a culture of the methanotrophic bacterium, a fermentation product of the methanotrophic bacterium, or a combination of two or more thereof.

10. Use of a methanotrophic bacterium, wherein the activity of malate thiokinase is enhanced, as compared to its endogenous activity, in producing PHB.
